# EUROPÄISCHE PATENTSCHRIFT

(11) **EP 2 523 703 B1**
(45) Veröffentlichungstag und Bekanntmachung des Hinweises auf die Patenterteilung: **18.03.2015**
(21) Anmeldenummer: 11700620.5
(22) Anmeldetag: 04.01.2011
(51) Int. Cl.: A61M 1/16, B01D 63/02, F28F 21/06

(54) **WÄRMETAUSCHERKÖRPER**
HEAT-EXCHANGER BODY
CORPS D'ÉCHANGEUR DE CHALEUR

(30) Priorität: 12.01.2010 DE 102010000820
(43) Veröffentlichungstag der Anmeldung: 21.11.2012
(73) Patentinhaber: RAUMEDIC AG, 95213 Münchberg (DE)
(72) Erfinder: STÖCKER, Martin, 95233 Helmbrechts (DE)
(74) Vertreter: Hofmann, Matthias
(86) Internationale Anmeldenummer: PCT/EP2011/050051
(87) Internationale Veröffentlichungsnummer: WO 2011/086010

(56) Entgegenhaltungen:
- DE-A1- 2 825 065
- DE-A1- 4 308 850
- US-A- 5 706 889
- US-A- 5 876 667

## Beschreibung

Die Erfindung betrifft einen Wärmetauscher eines Oxygenators. Ein Oxygenator ist ein medizinisches Produkt, das Blut mit Sauerstoff anreichert und Kohlendioxid aus dem Blut entfernt. Der Oxygenator wird in der Herzchirurgie als Teil der Herz-Lungen-Maschine verwendet und ersetzt beispielsweise während einer Operation am offenen Herzen kurzzeitig die Funktion der Lunge. An dem Oxygenator ist zusätzlich ein Wärmetauscher für einen Wärmeaustausch zwischen Blut und einem Wärmetauschermedium, insbesondere Wasser, vorgesehen.

Ein derartiger Oxygenator ist aus der US 5,706,889, der US 5,876,667, der DE 28 25 065 A1 und der DE 689 25 291 T2 bekannt. Die US 4,336,138 offenbart ein Verfahren zum Wickeln von Fasern auf einen Wickelkern. Die DE 43 08 850 A1 offenbart ein Verfahren zur Herstellung einer Hohlfadenmatte.

Die Wärmeübertragung zwischen dem Blut und dem Wärmetauschermedium erfolgt mittels eines Wärmetauscherkörpers, der entweder metallisch, insbesondere aus Edelstahl oder einer Aluminium-Verbindung, oder in Form eines Kunststoffbauteils, beispielsweise mit Polyester (PES)/Polyethylenterephthalat (PET)-Fasern ausgeführt ist. Es ist nachteilig, dass die genannten Materialien eine geringe Blutkompatibilität aufweisen. Darüber hinaus kann eine elektrostatische Aufladung des Kunststoff-Fasermaterials einen elektrischen Durchschlag verursachen, der zu einer Zerstörung des Wärmetauscherkörpers führen kann. Insbesondere gegenüber dem metallischen Werkstoff ist das Wärmetauschverhalten des Kunststoffmaterials reduziert, so dass ein Wärmetauscherkörper mit vergleichbarer Wärmetauschkapazität eine große Bauweise erfordert. Daraus erwachsen zusätzliche Probleme, beispielsweise wegen der notwendigen großen Kontaktfläche zwischen dem bedingt blutkompatiblen Kunststoffmaterial und dem Blut, hinsichtlich eines möglichen Druckabfalls in dem Oxygenator wegen der großen Baulänge, wobei der Druckabfall blutschädigend wirken kann, und hinsichtlich eines erforderlichen hohen Füllvolumens des Wärmetauschers, so dass zusätzliches Fremdblut oder weitere Medien wie eine Salzlösung notwendig werden, um den Oxygenator zu füllen.

Die aus dem Stand der Technik bekannten Wärmetauscher können zu Problemen bei Einsatz des Wärmetauschers während einer Operation führen.

Es ist eine Aufgabe der vorliegenden Erfindung, einen Wärmetauscher zu schaffen, der einfach aufgebaut ist und eine hohe Blutkompatibilität bei gleichzeitig gutem Wärmetauschverhalten gewährleistet.

Diese Aufgabe ist gelöst durch einen Wärmetauscher mit den im Anspruch 1 angegebenen Merkmalen.

Erfindungsgemäß wurde erkannt, dass ein Wärmetauscher mit einem Wärmetauscherkörper in Form einer Matte mit mehreren, jeweils eine Längsachse aufweisenden Kunststoffschlauchabschnitten, die zu einer Rolle gewickelt ist, ein verbessertes Wärmeübertragungsverhalten zwischen dem Blut und dem Wärmetauschermedium ermöglicht. Dazu ist die aus der Matte gewickelte Rolle des Wärmetauscherkörpers in einem eine Mittellängsachse aufweisenden rohrförmigen Grundkörper derart angeordnet, dass die Längsachsen der Kunststoffschlauchabschnitte parallel zu der Mittellängsachse orientiert sind. Weiterhin weist der Wärmetauscherkörper benachbart zu den Kunststoffschlauchabschnitten Blutströmungskanäle mit jeweils einer Blut-Einströmöffnung und einer Blut-Ausströmöffnung auf. Es kann auch genau ein derartiger Blutströmungskanal vorgesehen sein, wobei dann genau eine Blut-Einströmöffnung und genau eine Blut-Ausströmöffnung vorhanden sind.

Weiterhin wurde erkannt, dass ein Wärmetauscherkörper in Form einer Matte mit mehreren, jeweils eine Längsachse aufweisenden Kunststoffschlauchabschnitten zu einem verbesserten Wärmeübertragungsverhalten zwischen dem Blut und dem Wärmetauschermedium führt. Dazu sind die Kunststoffschlauchabschnitte mittels quer zur Längsachse verlaufender Halteelemente miteinander verbunden, so dass die Kunststoffschlauchabschnitte mit definierter Lage zueinander als Teil der Matte angeordnet sind und dadurch ein verbessertes Strömungsverhalten des durchströmenden Bluts und Wärmetauschermediums ermöglicht wird. Dazu weisen die Kunststoffschlauchabschnitte jeweils eine Eintrittsöffnung zum Eintritt und eine Austrittsöffnung zum Austritt des Wärmetauschermediums auf. Die Ausführung eines Wärmetauscherkörpers mit Kunststoffschlauchabschnitten aus Polyurethan (PUR) führt zu einer Verbesserung der Blutkompatibilität. Darüber hinaus wird eine elektrostatische Aufladung der Kunststoffschlauchabschnitte im Vergleich zu anderen Kunststoffmaterialien reduziert, so dass ein elektrischer Durchschlag und eine damit verbundene Zerstörung des Wärmetauscherkörpers weitestgehend vermieden werden kann. Gegenüber anderen Kunststoffmaterialien weist Polyurethan (PUR) ein verbessertes Wärmetauschverhalten auf und ermöglicht somit eine vergleichsweise kürzere Bauweise des Wärmetauscherkörpers, woraus sich weitere Vorteile ergeben. Insbesondere wird dadurch die mit dem Blut in Kontakt tretende Oberfläche verringert, so dass insgesamt die Blutkompatibilität verbessert wird. Der Druckabfall in dem Wärmetauscherkörper wird infolge der kürzeren Baulänge reduziert und wirkt sich damit schonend auf das in dem Wärmetauscher befindliche Blut aus. Weiterhin wird aufgrund der geringeren Baugröße weniger Füllvolumen für den Wärmetauschkörper erforderlich, so dass der Bedarf an Fremdblut bzw. einem weiteren Medium, wie beispielsweise einer Salzlösung, reduziert werden kann.

Bei einem Wärmetauscher nach Anspruch 2 sind die Blutströmungskanäle einfach und direkt durch das Wickeln der Matte zu der Rolle gebildet. Weiterhin führen die zwischen den Kunststoffschlauchabschnitten angeordneten Hohlräume, die die Blutströmungskanäle darstellen, zu einer Vergrößerung der Kontaktfläche zwischen dem in den Kunststoffschlauchabschnitten geführten Wärmetauschermedium und dem Blut, so dass die Wärmeübertragung zusätzlich verbessert ist.

Dadurch, dass das Blut bei einem Wärmetauscher nach Anspruch 3 an einer äußeren Mantelfläche der Kunststoffschlauchabschnitte strömt, ist eine äußere Wärmetauscherfläche für das Blut größer als eine innere Wärmetauscherfläche des Wärmetauschermediums innerhalb der Kunststoffschlauchabschnitte, wobei das Blut und das Wärmetauschermedium über eine Wärmedurchgangswand der Kunststoffschlauchabschnitte voneinander getrennt sind. Dadurch wird insbesondere ein Vermischen des Bluts mit dem Wärmetauschermedium vermieden.

Die Gestaltung eines Wärmetauschers nach Anspruch 4 ermöglicht eine kompakte und unverlierbare Gestaltung des Wärmetauschers, wobei der Wärmetauscherkörper durch das Umgießen von dem Grundkörper zusätzlich stabilisiert ist und ein unbeabsichtigtes Lösen der Rolle vermieden wird.

Ein Wärmetauscher mit einem Wärmetauscherkörper gemäß Anspruch 5 garantiert eine sichere Verbindung der Kunststoffschlauchabschnitte durch Wirknähte, wobei die Querschnittsform eines Kunststoffschlauchabschnitts im Bereich der Wirknaht durch diese nicht oder nur unwesentlich verändert ist, so dass die Durchströmung mit dem Wärmetauschermedium ungehindert erfolgen kann.

Bei einem Wärmetauscher mit einem Wärmetauscherkörper gemäß Anspruch 6 sind die Strömungsbedingungen für das durchströmende Wärmetauschermedium zusätzlich verbessert.

Eine Matte mit mehreren miteinander verwirkten Kunststoffschlauchabschnitten kann aus einem quasi endlosen Kunststoffschlauch hergestellt werden. Dazu wird der Kunststoffschlauch beispielsweise durch ein separates Zuführgerät bereitgestellt. Anschließend erfolgt ein mäanderförmiges Anordnen des Kunststoffschlauches mit mehreren parallel zueinander angeordneten Kunststoffschlauchabschnitten und die Kunststoffschlauchabschnitte verbindenden Verbindungsabschnitten. Abschließend werden die Kunststoffschlauchabschnitte zu einer Matte verwirkt. Das Verwirken kann mittels einer speziellen Verwirkmaschine erfolgen und ist insbesondere automatisiert durchführbar. Dadurch ist das Verfahren insgesamt zu einem sehr hohen Grad automatisierbar und damit kostengünstig durchführbar. Die Größe der herzustellenden Matte kann durch die Anzahl der nebeneinander anzuordnenden Kunststoffschlauchabschnitte eingestellt werden. Auch die Länge der Kunststoffschlauchabschnitte sowie die Abstände zwischen einzelnen Wirknähten sind in Abhängigkeit der herzustellenden Matte einstellbar. Bei dem Verfahren erfolgt ein Verschließen von Enden der Kunststoffschlauchabschnitte. Das Verschließen ist durch Knickstellen der Verbindungsabschnitte gewährleistet. Das Verfahren ermöglicht ein Verschließen der Kunststoffschlauchabschnitte während der Herstellung der Matte, so dass insbesondere eine Verunreinigung der Kunststoffschlauchabschnitte während des Herstellungsprozesses vermieden wird.

Ein Ausführungsbeispiel der Erfindung wird nachfolgend anhand der Zeichnung näher erläutert. In dieser zeigen:
- Fig. 1: eine schematische Ansicht eines erfindungsgemäßen Wärmetauscherkörpers in Form einer Matte von verwirkten Kunststoffschlauchabschnitten,
- Fig. 2: eine schematische Seitenansicht eines erfindungsgemäßen Wärmetauschers,
- Fig. 3: einen Schnitt gemäß Linie III-III in Fig. 2 und
- Fig. 4: eine vergrößerte Querschnittdarstellung eines Kunststoffschlauchabschnitts.

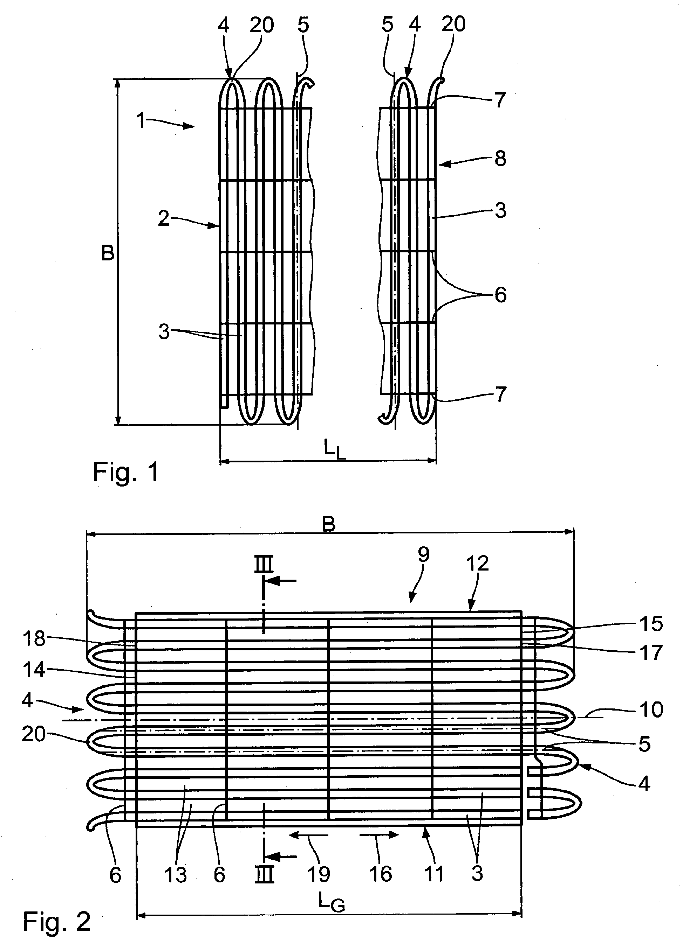

Ein in Fig. 1 dargestellter Wärmetauscherkörper 1 weist einen Kunststoffschlauch 2 aus Polyurethan (PUR) auf. Der Kunststoffschlauch 2 ist mäanderförmig angeordnet mit mehreren, parallel zueinander angeordneten Kunststoffschlauchabschnitten 3 und jeweils zwei Kunststoffschlauchabschnitte 3 verbindende Verbindungsabschnitten 4. Durch das mäanderförmige Verlegen des Kunststoffschlauches 2 weisen die Verbindungsabschnitte 4 eine Knickstelle 20 auf. Hier ist der Kunststoffschlauch 2 derart zusammengedrückt ist, dass die über den Verbindungsabschnitt 4 miteinander verbundenen Kunststoffschlauchabschnitten 3 hermetisch voneinander getrennt sind. Die Kunststoffschlauchabschnitte 3 weisen jeweils eine Längsachse 5 auf und sind mittels quer zu den Längsachsen 5 verlaufenden Halteelementen in Form von Wirknähten 6 miteinander verbunden.

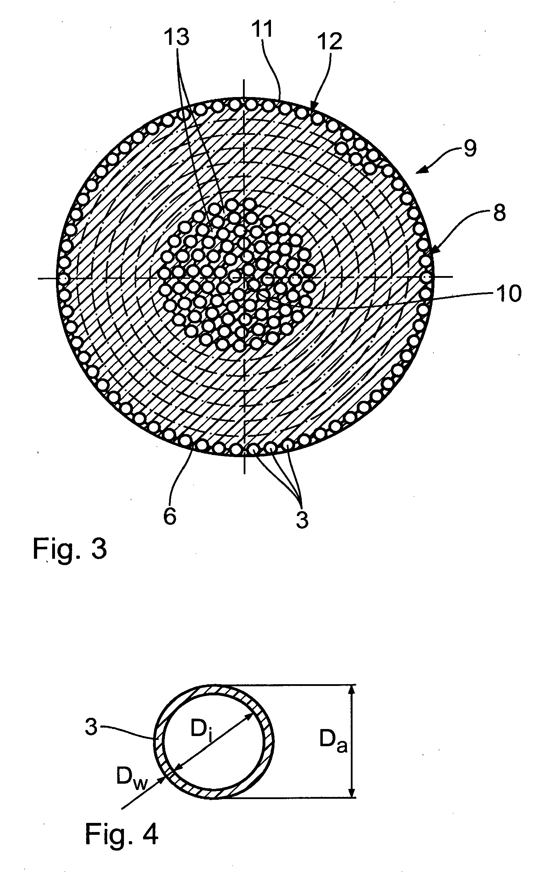

In dem in Fig. 4 gezeigten Ausführungsbeispiel weist der PUR-Kunststoffschlauch 2 einen Innendurchmesser Dᵢ von 0,67 mm und einen Außendurchmesser Dₐ von 0,79 mm auf. Daraus folgt, dass eine Wärmedurchgangswand eine Dicke D_{w} in Wärmedurchgangsrichtung durch die Schlauchwand von 0,06 mm aufweist. Es ist auch möglich einen Innendurchmesser Dᵢ von 0,50 mm und einen Außendurchmesser Dₐ von 0,60 mm für den PUR-Kunststoffschlauch 2 zu wählen. Ein Schlauch mit derartigen Abmessungen hat sich als besonders vorteilhaft hinsichtlich der Druckbeständigkeit erwiesen. In diesem Fall hat die Wärmedurchgangswand eine Dicke D_{w} in Wärmedurchgangsrichtung durch die Schlauchwand von 0,05 mm.

Die Kunststoffschlauchabschnitte 3 sind mittels der Wirknähte 6 zu einer Matte 8 verwirkt, wobei die Matte 8 eine Breite B von 170 mm ± 5 mm aufweist. Eine Lauflänge L_{L} der Matte 8 beträgt in dem gezeigten Ausführungsbeispiel 2 m, so dass etwa 250 m des PUR-Kunststoffschlauchs 2 zur Herstellung der Matte 8 verarbeitet worden sind.

Eine innere Wärmetauscherfläche des Wärmetauschermediums, insbesondere Wasser, wird von einer inneren Mantelfläche eines Kunststoffschlauchabschnitts 3 gebildet und ist damit kleiner als eine äußere Wärmetauscherfläche des Bluts, die von einer äußeren Mantelfläche des Kunststoffschlauchabschnitts 3 gebildet wird. In dem gezeigten Beispiel der Matte 8 ergibt sich aus der Gesamtzahl der Kunststoffschlauchabschnitte 3 eine innere Gesamt-Wärmetauscherfläche des Wärmetauschermediums von etwa 0,5 m² und eine äußere Gesamt-Wärmetauscherfläche des Bluts von etwa 0,6 m², so dass aufgrund der Vielzahl der Kunststoffschlauchabschnitte 3 eine insgesamt große Wärmetauscherfläche mit dem erfindungsgemäßen Wärmetauscherkörper 1 zur Verfügung gestellt wird, obwohl die einzelnen Kunststoffschlauchabschnitte 3 geringe Wärmetauscherflächen aufweisen.

In Fig. 2 ist eine Seitenansicht eines erfindungsgemäßen Wärmetauschers 9 dargestellt, der einen eine Mittellängsachse 10 aufweisenden rohrförmigen Grundkörper 11 und den in dem Grundkörper 11 angeordneten Wärmetauscherkörper 1 umfasst. Dazu ist die Matte 8 des Wärmetauscherkörpers 1 zu einer Rolle 12 gewickelt und derart in dem Grundkörper 11 angeordnet, dass die Längsachsen 5 der Kunststoffschlauchabschnitte 3 parallel zu der Mittellängsachse 10 des Grundkörpers 11 orientiert sind. Durch das Wickeln der Matte 8 zu der Rolle 12 weist der Wärmetauscherkörper 1 benachbart zu den Kunststoffschlauchabschnitten 3 Blutströmungskanäle 13 mit jeweils einer Blut-Einströmöffnung 14 und einer Blut-Ausströmöffnung 15 auf. Dabei sind die Blutströmungskanäle in Form von parallel zur Mittellängsachse 10 orientierten Hohlräumen 13 zwischen den Kunststoffschlauchabschnitten 3 der Rolle 12 ausgeführt. Durch die Anordnung der Blut-Einströmöffnung 14 an einem Ende des Hohlraums 13 und der Blut-Ausströmöffnung 15 an einem diesem Ende gegenüberliegenden Ende des Hohlraums 13 ist eine Blut-Strömungsrichtung 16 entlang der Mittellängsachse 10 des Grundkörpers 11 festgelegt.

Entsprechend weisen die Kunststoffschlauchabschnitte 3 jeweils eine Eintrittsöffnung 17 zum Eintritt des Wärmetauschermediums und eine Austrittsöffnung 18 zum Austritt des Wärmetauschermediums auf, wobei durch die Anordnung der Eintrittsöffnungen 17 und Austrittsöffnungen 18 an dem Wärmetauscher 9 eine Wärmetauschermedium-Strömungsrichtung 19 festgelegt ist. Die Wärmetauschermedium-Strömungsrichtung 19 ist entgegen der Blut-Strömungsrichtung 16 orientiert. Das bedeutet, dass der in Fig. 2 gezeigte Wärmetauscher im Gegenstromverfahren betrieben wird, so dass der Wirkungsgrad des Wärmetauschers 9 zusätzlich verbessert ist. Es ist auch möglich, den Wärmetauscher 9 im Gleichstromverfahren zu betreiben, so dass die Blut-Strömungsrichtung 16 und die Wärmetauschermedium-Strömungsrichtung 19 gleichgerichtet sind.

Wie in Fig. 2 gezeigt, ist die Breite B der Matte 8, die identisch ist mit der Breite B der Rolle 12, größer als eine Länge L_{G} des Grundkörpers 11 und damit des Wärmetauschers 9. Die Anpassung der Breite B der Rolle 12 an die Länge L_{G} des Grundkörpers 11 erfolgt durch beidseitiges Abtrennen der überstehenden Verbindungsabschnitte 4 der Rolle 12. Besonders vorteilhaft ist ein Abtrennen der Verbindungsabschnitte 4 derart, dass die verbleibende Rolle 12 an den Enden 7 der Kunststoffschlauchabschnitte 3 jeweils eine Wirknaht 6 aufweisen.

Bei dem in Fig. 2 gezeigten Wärmetauscher 9 ist der zu der Rolle 12 gewickelte Wärmetauscherkörper 1 mit dem Grundkörper 11 durch Umgießen fest verbunden. Der Grundkörper 11 ist dabei aus einem transparenten Kunststoff hergestellt.

Fig. 3 zeigt eine Schnittdarstellung des Wärmetauschers 9 mit dem Grundkörper 11 und der Rolle 12, wobei insbesondere die spiralförmige Anordnung der zu der Rolle 12 aufgewickelten Matte 8 erkennbar ist. Durch das Verwirken der einzelnen Kunststoffschlauchabschnitte 3 mittels der Wirknähte 6 entsteht in der Schnittansicht gemäß Fig. 3 eine perlenkettenförmige Anordnung der Matte 8. Infolge des losen Aufwickelns zu der Rolle 12 sind zwischen den Kunststoffschlauchabschnitten 3 die entsprechenden Hohlräume 13 gebildet.

Im Folgenden wird ein Verfahren zur Herstellung des Wärmetauscherkörpers 1 für den Wärmetauscher 9 eines Oxygenators näher beschrieben. Zunächst erfolgt ein Bereitstellen des Kunststoffschlauchs 2 durch ein aktives Zuführgerät. Dadurch wird die Zuführung des zu verwirkenden PUR-Schlauches 2 vorspannungsfrei ermöglicht und damit verhindert, dass ein Abrollen des Schlauches 2 von einer Spule zu einem Rutschen oder Verklemmen verschiedener Schlauchlagen und damit zu Vorspannungen in dem zugeführten Schlauchstrang führt. Dadurch könnte ein nachfolgendes Anordnen des Kunststoffschlauches 2 negativ beeinträchtigt werden. Das Anordnen des Kunststoffschlauches 2 erfolgt mäanderförmig mit mehreren parallel zueinander angeordneten Kunststoffschlauchabschnitten 3 und die Kunststoffschlauchabschnitte 3 verbindenden Verbindungsabschnitten 4, wie in Fig. 1 gezeigt. In dieser mäanderförmigen Anordnung wird der Kunststoffschlauch 2 mittels mehrerer quer zu den Längsachsen 5 der Kunststoffschlauchabschnitte 3 angeordneter Wirknähte 6 zu der Matte 8 durch eine Wirkmaschine verwirkt. In dem in Fig. 1 gezeigten Ausführungsbeispiel der Matte 8 sind fünf Wirknähte 6 vorgesehen. Es ist auch möglich, mehrere, insbesondere neun Wirknähte 6 vorzusehen, wobei jeweils zwei äußere Wirknähte 6 an den Enden 7 der Kunststoffschlauchabschnitte 3 vorgesehen sind. Die übrigen Wirknähte 6 sind vorzugsweise in regelmäßigen Abständen entlang der Längsachsen 5 zwischen den beiden äußeren Wirknähten 6 angeordnet. Ein bevorzugter Abstand zwischen zwei benachbarten Wirknähten beträgt in etwa 2 cm.

Nach dem Verwirken der Kunststoffschlauchabschnitte 3 zu der Matte 8 sind die Kunststoffschlauchabschnitte 3 für das weitere Herstellungsverfahren durch die Knickstellen 20 in den Verbindungsabschnitten 4 hermetisch abgedichtet und somit insbesondere vor Verunreinigungen während des weiteren Herstellungsprozesses geschützt. Dies ist eine hygienische Voraussetzung für die spätere Verwendung des Wärmetauscherkörpers 1 in dem Wärmetauscher 9 für einen Oxygenator. In diesem Zustand weist die Matte 8 eine Breite B auf.

Anschließend wird die Matte 8 auf einen nicht dargestellten Kern aufgewickelt und durch Umgießen mit dem Grundkörper 11 fest verbunden. Wie in Fig. 2 dargestellt, ist die Breite B der Matte 8 größer als die Länge L_{G} des Grundkörpers 11, so dass die beidseitig überstehenden Verbindungsabschnitte 4 in einem weiteren Fertigungsschritt von dem innerhalb des Grundkörpers 11 angeordneten Wärmetauscherkörper 1 getrennt werden. Dazu erfolgt zunächst ein Abtrennen der Verbindungsabschnitte 4 und anschließend ein Verschweißen der durch das Abtrennen geöffneten Enden 7 der Kunststoffschlauchabschnitte 3. Sowohl das Abtrennen als auch das Verschweißen erfolgt bei dem gezeigten Ausführungsbeispiel durch thermische Materialbearbeitung, wobei auch andere Verfahren zum Einsatz kommen können. Vor der Verwendung des erfindungsgemäßen Wärmetauschers 9, werden die Kunststoffschlauchabschnitte 3 des Wärmetauscherkörpers 1 wieder geöffnet und dadurch die Blut-Einströmöffnungen 14, die Blut-Ausströmöffnungen 15, die Eintrittsöffnungen 17 und die Austrittsöffnungen 18 gebildet, so dass der Wärmetauscher 9 zum Durchströmen mit Blut und dem Wärmetauschermedium, wofür bevorzugt Wasser eingesetzt wird, genutzt werden kann.

## Patentansprüche

1. Wärmetauscher für einen Oxygenator, umfassend
a. einen Grundkörper (11),
b. einen in dem Grundkörper (11) angeordneten Wärmetauscherkörper (1) mit einer Matte (8) mit mehreren, jeweils eine Längsachse (5) aufweisenden Kunststoffschlauchabschnitten (3),
i. die mittels quer zur Längsachse (5) verlaufenden Halteelementen (6) miteinander verbunden sind,
ii. die eine Eintrittsöffnung (17) zum Eintritt von Wärmetauschermedium aufweisen,
iii. die eine Austrittsöffnung (18) zum Austritt des Wärmetauschermediums aufweisen und
iv. die Polyurethan aufweisen,
c. wobei der Wärmetauscherkörper (1) benachbart zu den Kunststoffschlauchabschnitten (3) Blutströmungskanäle (13) mit jeweils einer Blut-Einströmöffnung (14) und einer Blut-Ausströmöffnung (15) aufweist,
d. wobei durch die Anordnung der Eintrittsöffnungen (17) und Austrittsöffnungen (18) an dem Wärmetauscher (9) eine Wärmetauschermedium-Strömungsrichtung (19) festgelegt ist,
e. wobei durch die Anordnung der Blut-Einströmöffnungen (14) an einem Ende der Blutströmungskanäle (13) und Blut-Ausströmöffnungen (15) an einem diesem Ende gegenüberliegenden Ende der Blutströmungskanäle (13) eine Blut-Strömungsrichtung (16) festgelegt ist,
**dadurch gekennzeichnet, dass**
f. der Grundkörper (11) rohrförmig ausgeführt ist und eine Mittellängsachse (10) aufweist,
g. die Matte (8) des Wärmetauscherkörpers (1) zu einer Rolle (12) gewickelt ist,
h. die Längsachsen (5) der Kunststoffschlauchabschnitte (3) parallel zu der Mittellängsachse (10) orientiert sind,
i. die Wärmetauschermedium-Strömungsrichtung (19) entgegen der Blut-Strömungsrichtung (16) orientiert ist,
j. die Blut-Strömungsrichtung (16) entlang der Mittellängsachse (10) des Grundkörpers (11) orientiert ist.

2. Wärmetauscher nach Anspruch 1, **gekennzeichnet durch** parallel zur Mittellängsachse (10) orientierte Hohlräume (13) zwischen den Kunststoffschlauchabschnitten (3) der Rolle (12) als Blutströmungskanäle.

3. Wärmetauscher nach Anspruch 1 oder 2, **dadurch gekennzeichnet, dass** jeder Kunststoffschlauchabschnitt (3) eine Wärmedurchgangswand aufweist, wobei eine innere Wärmetauscherfläche des Wärmetauschermediums kleiner ist als eine äußere Wärmetauscherfläche des Bluts.

4. Wärmetauscher nach einem der Ansprüche 1 bis 3, **dadurch gekennzeichnet, dass** der Grundkörper (11) mit dem Wärmetauscherkörper (1), insbesondere durch Umgießen, fest verbunden ist.

5. Wärmetauscher nach einem der vorangegangenen Ansprüche, **dadurch gekennzeichnet, dass** die Kunststoffschlauchabschnitte (3) quer zur Längsachse (5) durch Wirknähte (6) miteinander verwirkt sind.

6. Wärmetauscher nach einem der vorangegangenen Ansprüche, **dadurch gekennzeichnet, dass** die Längsachsen (5) der Kunststoffschlauchabschnitte (3) parallel zueinander angeordnet sind.

## Claims

1. Heat exchanger for an oxygenator, comprising
a. a base body (11),
b. a heat exchanger body (1) arranged in the base body (11), said heat exchanger body (1) comprising a mat (8) with several plastic tube portions (3) each having a longitudinal axis (5), said plastic tube portions (3)
i. being interconnected by means of retaining members (6) running transversely to the longitudinal axis (5),
ii. having an inlet opening (17) allowing a heat exchanger medium to enter,
iii. having a discharge opening (18) allowing the heat exchanger medium to be discharged, and
iv. containing polyurethane,
c. wherein the heat exchanger body (1) has blood flow passages (13) adjacent to the plastic tube portions (3), each of said blood flow passages (13) comprising a blood inlet opening (14) and a blood discharge opening (15),
d. wherein a direction of heat exchanger medium flow (19) is defined by the arrangement of the inlet openings (17) and discharge openings (18) at the heat exchanger (9),
e. wherein a direction of blood flow (16) is defined by the arrangement of the blood inlet openings (14) at one end of the blood flow passages (13) and blood discharge openings (15) at an end of the blood flow passages (13) opposite to said one end,
**characterised in that**
f. the base body (11) has a tubular shape and has a central longitudinal axis (10),
g. the mat (8) of the heat exchanger body (1) is rolled up into a roll (12),
h. the longitudinal axes (5) of the plastic tube portions (3) are oriented parallel to the central longitudinal axis (10),
i. the direction of heat exchanger medium flow (19) is oriented opposite to the direction of blood flow (16),
j. the direction of blood flow (16) is oriented along the central longitudinal axis (10) of the base body (11).

2. Heat exchanger according to claim 1, **characterised by** hollow spaces (13) oriented parallel to the central longitudinal axis (10) between the plastic tube portions (3) of the roll (12), said hollow spaces (13) serving as blood flow passages.

3. Heat exchanger according to claim 1 or 2, **characterised in that** each plastic tube portion (3) has a heat transfer wall, wherein an inner heat exchanger surface area of the heat exchanger medium is smaller than an outer heat exchanger surface area of the blood.

4. Heat exchanger according to one of claims 1 to 3, **characterised in that** the base body (11) is rigidly connected to the heat exchanger body (1), in particular by casting said base body (11) around said heat exchanger body (1).

5. Heat exchanger according to one of the preceding claims, **characterised in that** the plastic tube portions (3) are interwoven in a direction transverse to the longitudinal axis (5) by means of weaving seams (6).

6. Heat exchanger according to one of the preceding claims, **characterised in that** the longitudinal axes (5) of the plastic tube portions (3) are arranged parallel to each other.

## Revendications

1. Echangeur de chaleur destiné à un oxygénateur, comprenant
a. un corps de base (11),
b. un corps échangeur de chaleur (1), disposé dans le corps de base (11), doté d'une natte (8) avec plusieurs sections de tuyaux en matière synthétique (3), présentant chacune un axe longitudinal (5),
i. qui sont reliées ensemble au moyen d'un élément de fixation (6) s'étendant perpendiculairement par rapport à l'axe longitudinal (5),
ii. qui présentent un orifice d'entrée (17) pour l'entrée du fluide échangeur de chaleur,
iii. qui présentent un orifice de sortie (18) pour la sortie du fluide échangeur de chaleur, et
iv. contiennent du polyuréthane,
c. le corps échangeur de chaleur (1) présentant des canaux d'écoulement pour le sang (13) avec chacun un orifice d'entrée pour l'écoulement du sang (14) et un orifice de sortie pour l'écoulement du sang (15) au voisinage des sections de tuyau en matière synthétique (3),
d. un sens d'écoulement du fluide échangeur de chaleur (19) étant réalisé par l'intermédiaire de l'agencement des orifices d'entrée (17) et des orifices de sortie (18) sur l'échangeur de chaleur (9),
e. un sens d'écoulement du sang (16) étant réalisé par l'agencement des orifices d'entrée pour l'écoulement du sang (14) à une extrémité des canaux d'écoulement du sang (13) et des orifices de sortie pour l'écoulement du sang (15) à l'une des extrémités des canaux d'écoulement du sang (13) située en face de cette extrémité,
**caractérisé en ce que**
f. le corps de base (11) est conçu sous la forme d'un tuyau et présente un axe longitudinal central (10),
g. la natte (8) du corps échangeur de chaleur (1) est enroulée comme une bobine (12),
h. les axes longitudinaux (5) des sections de tuyaux en matière synthétique (3) sont orientés parallèlement par rapport à l'axe longitudinal central (10),
i. le sens d'écoulement du fluide échangeur de chaleur (19) est orienté en sens inverse du sens d'écoulement du sang (16),
j. le sens d'écoulement du sang (16) est orienté le long de l'axe longitudinal central (10) du corps de base (11).

2. Echangeur de chaleur selon la revendication 1, **caractérisé par** des corps creux (13) orientés parallèlement par rapport à l'axe longitudinal central (10) entre les sections de tuyau en matière synthétique (3) de la bobine (12) servant de canaux d'écoulement du sang.

3. Echangeur de chaleur selon les revendications 1 ou 2, **caractérisé en ce que** chaque section de tuyau en matière synthétique (3) présente une paroi permettant le passage de la chaleur, où une surface de l'échangeur de chaleur interne du fluide échangeur de chaleur est inférieure à une surface de l'échangeur de chaleur externe correspondant au sang.

4. Echangeur de chaleur selon l'une des revendications 1 à 3, **caractérisé en ce que** le corps de base (11) est solidement relié, notamment par la pose d'un cordon fondu, avec le corps échangeur de chaleur (1).

5. Echangeur de chaleur selon l'une des revendications précédentes, **caractérisé en ce que** les sections de tuyau en matière synthétique (3) sont liées inextricablement ensemble par des sutures actives (6) perpendiculaires par rapport à l'axe longitudinal (5).

6. Echangeur de chaleur selon l'une des revendications précédentes, **caractérisé en ce que** les axes longitudinaux (5) des sections de tuyau en matière synthétique (3) sont disposés parallèles les uns par rapport aux autres.
